# EUROPEAN PATENT APPLICATION

(11) **EP 4 763 055 A1**
(43) Date of publication of application: **24.06.2026**
(21) Application number: 25157487.7
(22) Date of filing: 12.02.2025
(51) Int. Cl.: A61B 5/00, A61N 1/05, A61B 5/293, A61M 25/00

(54) **MULTI-CONTACT INTRACEREBRAL FUNCTIONAL EXPLORATION PROBE WITH AN INTEGRATED DRUG DELIVERY SYSTEM**

(30) Priority: 18.12.2024 US 202418985267
(71) Applicant: NEXT NEUROTECH, 30000 Nîmes (FR)
(72) Inventor: CABAUD, Pierre-Antoine, 25000 BESANÇON (FR); VALORGE, Yoann, 30100 ALÈS (FR); HAMMOUD, Abdul Karim, 1163 ETOY (CH)
(74) Representative: Cabinet Beau de Loménie

(57) **Abstract**

A multi-contact neural probe (10) for functional study and/or stimulation and/or treatment of a brain (131) by radiofrequency, comprising:
- an elongated tubular housing (100) with a proximal end (103), a distal end (101), and an internal lumen (25),
- a multi-layer film (5) arranged on an outer surface of said housing (100), said multi-layer film (5) comprising:
∘ a polymer substrate (20) and
∘ at least one conductive layer (10) deposited on said substrate (20) and including:
▪ one intracerebral contact (11) arranged on said distal end (101),
▪ at least one connecting contact (77) arranged on said proximal end (103) and intended to be connected to at least one external device (110), and
▪ at least one transmission track (12) connected to said at least one intracerebral contacts (11) and said connector contacts (77),

- at least one opening (120) arranged on said distal end (101) of said housing (100) and allowing a fluid communication between the lumen (25) and an aera facing said at least one opening (100), and
- a catheter (300) designed to transport a liquid drug and having an aperture (320) removably lodged inside the lumen (25) and movable inside the lumen (25) between a first position where said aperture (320) of the catheter (300) faces one of said at least one opening (120) of the housing (100) in order to deliver some liquid drug and a second position where said aperture (320) of the catheter (300) is blocked by a portion of a wall of the housing (100) in order to prevent any liquid drug delivery outside of the housing (100).

## Description

### Technical field

The present invention relates to the medical field, and in particular, to multi-contact neural probes for studying intracerebral function and allowing drug delivery in localised parts of the brain and methods of making such probes. The invention also relates to a device for functional studies and/or stimulation and/or treatment by radiofrequency in the brain, comprising such a probe.

### Background of the invention

To diagnose or treat certain conditions, such as drug-resistant epilepsy or Parkinson's disease, it can be useful to use intracerebral probes, which are often temporarily implanted in the patient's brain.

Such a probe can be implanted in a patient's brain to record electrical activity within the brain during a stereo electroencephalogram (SEEG), or to generate electrical stimulation and, if appropriate, identify abnormalities and then potentially treat the abnormality. Based on the hypothesis regarding the origin of the condition, intracerebral probes are implanted according to a previously developed implantation protocol for each patient.

Intracerebral probes are typically 5 cm to 15 cm long devices and, from the surgeon's perspective, are divided into three parts:
- the "distal" part, i.e. the part intended for implantation in the patient at a distance from the surgeon,
- the "proximal" part, i.e. the part near the surgeon intended to connect the probe to the recording or processing device and/or the signal transmission device, and
- an intermediate portion connecting the distal and proximal portions, and usually cylindrical.

The distal portion consists of alternating conductive areas connected by conductive tracks to connectors which are located in the proximal portion.

To manufacture the distal portion of the probe, it is known to use a tube of thermoplastic polymer material with a metal ring mounted thereon.

Another known manufacturing method involves juxtaposing a series of cylindrical sections each consisting of a thermoplastic polymer material and metal and welding or gluing them together.

In both cases, the number of conductive areas is limited by the robustness of the probe assembly and the number of cables to insert in a tube (1 cable per contact) with a bigger diameter (external tube). In fact, in order for these probes to be safe from a hygienic perspective, they must be strong enough to be removed without leaving foreign objects in the patient's brain. Therefore, the number of conductive areas is limited by the robustness of the probe assembly.

Therefore, there is a need for a multi-contact probe for intracerebral function research that is robust and reliable and can include a large number of conductive areas.

Further, for better treatment efficient there is also a need to be able to deliver drugs at the same time as the brain activity is recorded or at the same time and place that it is stimulated. And this while maintaining a robust and reliable neural probe with a large number of conductive areas.

### Object and summary of the invention

This need is fully or partially solved by the present invention, which in one aspect benefits from a multi-contact neural probe for functional studies and/or stimulation and/or treatment by radiofrequency in the brain, comprising:
- an elongated tubular housing with a proximal end, a distal end intended to be implanted inside a patient's brain, and an internal lumen forming a longitudinal passage inside the housing from the proximal end to the distal end,
- a multi-layer film arranged on an outer surface of said elongated tubular housing, said multi-layer film comprising:
   o a polymer substrate and
   o at least one conductive layer deposited on said substrate and including:
      ▪ one intracerebral contact arranged on said distal end,
      ▪ at least one connecting contact arranged on said proximal end and intended to be connected to at least one external device for recording and/or stimulating and/or treating, said at least one external device being external from a body of said patient, and
      ▪ at least one transmission track connected to said at least one intracerebral contacts and said connector contacts,
- at least one opening arranged on said distal end of said housing and allowing a fluid communication between the lumen and an aera facing said at least one opening, and
- a catheter designed to transport a liquid drug and having an aperture, said catheter being removably lodged inside the lumen and being movable inside the opening between a first position where said aperture of the catheter faces one of said at least one opening of the housing in order to deliver some liquid drug and a second position where said aperture of the catheter is blocked by a portion of a wall of the housing in order to prevent any liquid drug delivery outside of the housing.

The combination of the lumen formed inside the probe together with the removable catheter which can be moved, i.e. rotated and/or translated, inside the lumen of the housing provides the probe with an actionable drug delivery system. Thus, making the probe, a robust multi-contact neural probe with an actionable drug delivery system.

Indeed, as the catheter can be moved between two positions, the probe can be set in two different modes: a first one wherein the drug delivery system is closed, the aperture of the catheter and the opening of the housing not facing each other and therefore not communicating with each other so that the aperture of the catheter is blocked by the wall of the housing, preventing any liquid from flowing out of the catheter; and a second one wherein the drug delivery system is opened. In this second position, the aperture of the catheter is aligned with an opening of the housing, allowing a liquid to flow out of the catheter through the aperture of the catheter and the opening of the housing.

The polymer substrate of the multi-layer film includes at least one polymeric material and is preferably made in one piece. The substrate being manufactured in one piece, the distal part and the proximal part are obtained from the same substrate as well as an intermediate part extending between the proximate part and the distal part and which has a flat portion.

The at least one intracerebral contact of the multi-layer film is intended to be in contact with the patient's brain in order to obtain electrical signal characteristics of brain activity or to send electrical signals to areas of the patient's brain for treatment.

"At least one conductive layer" means at least one material layer with good electrical conductivity, such as a metal layer, graphite layer or any other material layer with good electrical conductivity, especially biocompatibility in areas prone to contact with the patient material layer. The at least one conductive layer may form only the wires constituting the transmission track, and in particular the intracerebral contacts and the connecting contacts at the ends of the transmission track.

The or each conductive layer may comprise gold and/or platinum and/or copper and/or iridium and/or any other biocompatible conductive material, preferably platinum and/or gold and/or copper.

Preferably, the or each conductive layer has a thickness of about 1 µm to 100 µm, preferably about 5 µm to 15 µm.

The multi-contact brain function research probe according to the present invention is reliable. In fact, given that the distal part of the probe consists of a limited number of parts that are fixed together, particularly single parts, the risk of the probe detaching during insertion or withdrawal is low.

The fact that the or each transmission track is connected to intracerebral contacts and connecting contacts enables transmission of one or more signals picked up by said intracerebral contacts. When multiple intracerebral contacts are present, each of them is preferably connected to a unique transmission track specific to it and associated with it, and each of them is also preferably connected to a unique transmission track specific to it and associated with it. It is associated with unique connecting contacts.

In a particular embodiment, the probe may comprise a tubular stiffener inserted between the housing and the catheter, fixed onto the housing, and which has an opening for each opening of said housing, the or each opening of the stiffener facing an opening of the housing. The tubular stiffener may extend along the distal end of the housing.

In another particular embodiment, the probe may comprise an end-tip, or distal stud, mounted on said distal end of the housing and closing said housing at said distal end. The end-tip can be a plug or a portion of the multi-layer film itself.

Said distal stud, or end-tip, may be made of at least one electrically conductive material, in particular at least one metal. In this case, the distal stud, when conducting electricity, is able to form an intracerebral contact that is independent of other intracerebral contacts. In this case, the probe advantageously includes an electrically conductive transmission track which, when electrically conductive, is in contact with the distal stud. The probe may alternatively include a transmission line connected to the distal stud, such as to connecting contacts on the proximal portion.

The tubular stiffener may comprise said end-tip, said end-tip being formed in the same material bloc as the tubular stiffener.

The lumen can also be at least partially filled with at least one glue or polymer material or composite material, in particular silicone filled with metal particles. The filling can be uniform or uneven.

When the internal cavity formed by the cylinder of the distal portion is at least partially filled with metal particle-filled silicone and the probe includes a conductive distal stud, the metal particle-filled silicone may enable the conductive distal end to be an electrical connection is established between the stud and a conductive transmission line or track in contact with the filled silicone.

Further, in a particular embodiment, an opening of said at least one opening of the housing may be arranged in the end-tip.

In a particular embodiment, said at least one opening arranged in the housing has an elongated shape along the longitudinal direction extending between the proximal end and the distal end.

In a particular embodiment, said at least one opening of said housing can be filled with a micro-porous material allowing a fluid to pass through. In a particular embodiment, the at least one opening of said housing can be made in the same polymer material as the rest of the housing and then a plurality of micro-holes can be made with a laser to design a micro-porous region in the place of the opening.

In a particular embodiment, the catheter may comprise a locking system on a proximal end to realize a coupling with a tank or another tubular system.

In a particular embodiment, said catheter can comprise an integrated tank.

In another particular embodiment, the tubular stiffener may extend only along the distal end of the housing.

The probe may include 1 to 500 intracerebral contacts, particularly 2 to 20 intracerebral contacts.

In a particular embodiment, the catheter may comprise at least one seal around its aperture on a radially outside face of the catheter

In a particular embodiment, the catheter may comprise a single aperture.

And in a variant, the catheter may comprise several apertures, at least two of said apertures being aligned along an axial direction of said catheter and sharing a same position along a circumferential direction, and said housing comprising two openings arranged to face said two apertures when the catheter is in its first position.

Preferably, the at least one transmission track is insulated from the patient's brain. The multilayer film may comprise at least one insulating layer of polymeric material, preferably liquid crystalline polymeric material, deposited on a substrate. In this case, the at least one insulating layer may at least partially cover the at least one transmission track.

The at least one insulating layer advantageously covers the at least one transmission track except for through holes to access the transmission track which are drilled and filled with a conductor to make a connecting pin allowing access to the transmission track through the insulating layer. In this way, the at least one intracerebral contact and the at least one transmission track are connected by way of through-holes or wells in the at least one insulating layer, which through-holes preferably transversely, in particular with the at least one The insulation layers extend orthogonally. Similarly, the at least one connecting contact and the at least one transmission track are connected by vias or wells in the at least one insulating layer, preferably laterally, in particular with the at least one insulating layer Extend orthogonally.

In another embodiment, the at least one insulating layer preferably does not cover the at least one intracerebral contact so that the at least one intracerebral contact is in contact with the brain and is capable of acquiring or transmitting electrical signals.

The at least one insulating layer may comprise a polymer material selected from the group consisting of liquid crystal polymer, polyamide, silicone and any other biocompatible thermoplastic polymer material, preferably a liquid crystal polymer material.

The one or more insulating layers are made of the same material as the substrate, for example.

One or more insulating layers may be deposited on the substrate and then fixed to the substrate by compressing and/or heating the one or more insulating layers.

If necessary, the thickness of the or each insulating layer is preferably about 1 µm to 1600 µm (inclusive), preferably about 20 µm to 50 µm (inclusive).

The substrate preferably includes at least one liquid crystal polymer (LCP) material.

A substrate including at least one liquid crystal polymer material can impart good long-term reliability to the probe. This enables, for example, the probe to be inserted into a patient's brain for days, or even months, with high-quality electrical signal transmission throughout the entire period.

The thickness of the substrate is preferably about 1 µm to 160 µm (inclusive), preferably about 25 µm to 90 µm (inclusive).

The distal part may comprise at least one temperature sensor formed in particular by said at least one electrically conductive layer. In this case, a multi-touch intracerebral functional probe can obtain thermal data about the patient's brain. The or each temperature sensor may be a resistance temperature sensor, such as a platinum resistance sensor. Temperature sensors may include thermocouples.

In one embodiment, each intracerebral contact extends around the entire circumference of the distal portion of the probe. Alternatively, the at least one intracerebral contact extends only over a portion of the circumference of the distal portion of the probe.

When the probe includes multiple intracerebral contacts, they may be spaced apart from each other, with pairs of adjacent intracerebral contacts separated by an intercontact distance that may be constant or varying. An insulating layer can be present in the space between two adjacent intracerebral contacts.

Deposited intracerebral contacts can be of the same or different lengths. "Length" refers to the length of the at least one intracerebral contact in a direction parallel to the longitudinal axis of the substrate.

The at least one intracerebral contact may comprise at least two separate parts. One or more intracerebral contacts may have a circular shape with the same or different radii.

Each intracerebral contact can be connected to a single connecting contact via a single transmission track. The connecting contacts enable connection with recording and/or stimulation and/or treatment devices external to the patient's body to transmit electrical signals in one direction and the other.

The proximal portion and/or the distal portion may extend along a longitudinal axis. Such a longitudinal axis may be straight or curved, or may include straight and curved portions. The longitudinal axis of the proximal part and/or the distal part may be the longitudinal axis of the substrate (especially when forming a cylinder constituting the proximal part and/or the distal part), but the orientation of the distal part and the proximal part is of course can vary (especially during use of the probe) and preferably can be moved relative to each other, in particular by means of connecting portions.

According to another aspect of the invention, independently of and/or in combination with the foregoing, it is also an object of the invention to provide a device for functional studies and/or stimulation and/or treatment of the brain, in particular by radiofrequency. A multi-contact probe as defined here above.

According to another aspect of the present invention, in conjunction with the above content, it is also a subject-matter of the present invention to provide a method for manufacturing a multi-contact neural probe as defined above for use in brain function research and/or stimulation and/or treatment, especially through radio frequency needle method, which includes the following steps:
a) step a: forming a multilayer film by flatly depositing at least one conductive layer forming at least one intracerebral contact, at least one connecting contact and at least one transmission track on at least a portion of the substrate, each transmission track being connected to intracerebral contacts and connecting contacts,
b) Step b: forming a hollow cylinder around a catheter, said hollow cylinder extending along a longitudinal axis from at least a first portion of the multilayer film to obtain a distal end of the probe intended for implantation in a brain of a patient to a second portion of the multilayer film to obtain the proximal end of the probe intended to be connected to at least one device for recording and/or stimulation and/or treatment outside the patient.

"Deposited flatly" means that during step a of depositing the one or more conductive layers, the substrate has a substantially flat shape.

A single substrate preferably made in one piece is used to form the distal end and the proximal end, and an intermediate portion extending between the distal end and the proximal end. Therefore, this method is capable of manufacturing a multi-contact intracerebral functional study probe with a limited number of parts fixed together, particularly a single part, which limits the insertion of the distal portion of the multi-contact intra-cerebral functional study probe. Risk of probe disintegration during removal from or in the patient's brain and limits the overall size of the probe.

Therefore, the distal end and the proximal end are preferably made out of a same material bloc, as well as an intermediate portion extending between the distal end and the proximal end.

The substrate is preferably made of at least one liquid crystal polymer (LCP) material or of polyimide. In fact, liquid crystal polymer materials are neither damaged nor dissolved by organic solvents used in microfabrication, such as alcohols, acetone, photoresins, photoresin developers/dissolvers or acid etchants for metals. This solvent resistance enables the at least one conductive layer to be deposited with high precision, for example by means of a centrifugal coating process, a metallization process, a photolithography process, or a dry or wet etching process.

Therefore, the use of a substrate made of at least one liquid crystal polymer material facilitates and improves the deposition of the at least one conductive layer.

Step a may comprise forming one or more layers on the substrate, in particular one or more conductive layers. To this end, step a may comprise producing a stack of different layers, in particular of different electrically conductive layers.

The at least one connecting contact is preferably formed on the second part.

Step a may include, after depositing the at least one conductive layer on the substrate, compressing and/or heating the at least one conductive layer on the substrate to fix the at least one conductive layer on the substrate.

The at least one electrically conductive layer is preferably produced separately and then deposited on the substrate.

The method may comprise the step of, in particular before step b, finishing the at least one electrically conductive layer, for example stripping and/or wet or dry etching.

It is preferred or even necessary to insulate the at least one transmission track from the patient's brain. Therefore, the method may comprise the step of depositing on the substrate before step b at least one insulating layer of polymeric material, said at least one insulating layer at least partially covering said at least one transport track. In this manner, each signal acquired by the or each intracerebral contact can be transmitted through the transmission track associated with the intracerebral contact without being transmitted between the transmission track and the patient's brain. Any electrical contact cuts or interferes with.

The flat substrate is preferably elongated along the longitudinal axis.

Step a may be performed such that the at least one intracerebral contact has a transverse width of about 0.1 mm to 10 mm (inclusive), preferably equal to about 2 mm, and a longitudinal length of about 0.1 mm to 10 mm (inclusive), preferably a surface equal to approximately 2mm.

"Lateral width" refers to the width of the at least one intracerebral contact in a direction transverse to the longitudinal axis of the substrate.

"Longitudinal length" refers to the length of the at least one intracerebral contact in a direction parallel to the longitudinal axis of the substrate.

The at least one intracerebral contact may extend over the entire or only part of the lateral width of the substrate.

When multiple intracerebral contacts are deposited on a substrate, they can be deposited at a distance from each other, with adjacent intracerebral contacts separated by an inter-contact distance that can be constant or varying. An insulating layer can be deposited at the spatial location between the two intracerebral contacts.

The at least one intracerebral contact may comprise at least two separate portions and extend from a lateral end of the substrate.

Intracerebral contacts can be arranged to obtain circular shapes with the same or different radii.

In a particular embodiment, during step a, at least one temperature sensor is deposited by gluing, welding or stacking on the substrate, preferably on said first part of the membrane. In this case, a multi-contact intracerebral functional probe can obtain thermal data from the patient's brain. The or each temperature sensor may be a resistive temperature sensor, such as one using a platinum resistor. Such a sensor may be formed from at least one conductive layer deposited during step a. Temperature sensors may include thermocouples.

The first part and/or the second part of the film may have two lateral edges. In this case, step b may comprise at least partially winding said first part and/or said second part of the film onto itself.

In one embodiment, said first portion and/or said second portion of the multilayer film includes two lateral edges, step b and/or step c includes placing said first portion and/or said second portion of said multilayer film Or the second portion is at least partially rolled around itself to at least partially overlap the lateral edges.

"Two lateral edges" means, for each edge, including a lateral end of the multilayer film and a portion of the surface of the multilayer film in the vicinity of the lateral end.

The lateral edges may be glued and/or welded together.

The substrate may comprise at least one window on at least one of said lateral edges, and step a can be performed to deposit said at least one conductive layer outside said at least one window. Fixing the edges together may be facilitated by the presence of one or more windows, the or each window increasing the fixed length between the two lateral edges.

In one or another of these embodiments, the first portion and/or the second portion of the multilayer film can be formed by sequentially placing the first portion and/or the second portion of the film. The diameter of the equivalent circular cross-section of the first and/or second portion of the membrane is inserted into at least one frusto-cone and is wound then decreases.

In another embodiment, the substrate has within its thickness an internal cavity that is laterally closed but open at least at one longitudinal end, step b comprising filling the substrate with at least one material, in particular at least one biocompatible material. of the internal cavity.

Advantageously, the method may comprise a step of inserting, in particular during or after step b, a distal stud at the distal end of the multilayer membrane, in particular an end-tip comprising at least one electrically conductive material, for example at least one metal studs.

Such distal studs may be secured by gluing, welding, moulding, over-moulding, and/or mechanical fixation (e.g., by forced insertion). Such a distal stud may project longitudinally from the distal end of the multilayer membrane and form the distal end of the distal portion. When the distal stud is at least partially made of metal, the distal stud advantageously forms an intracerebral contact independent of said at least one intracerebral contact deposited on the substrate during step a, that is to say distally

The device may be connected to a third party data processing device adapted to process signals acquired by one or more intracerebral contacts and transmitted by an associated transmission track or tracks.

One or more connecting contacts present on the proximal end enable the transmission of electrical signals from the probe to the recording and/or treatment and/or stimulation device for the patient and/or to the data processing device and vice versa. Of course.

This device makes it possible to obtain a diagnosis of brain activity, such as stereoelectroencephalography (SEEG), and/or treatment of one or more areas of the brain.

The device is capable of collecting data about a patient's neural activity.

The device can achieve electrical stimulation of the patient's brain, particularly in the vicinity of the at least one intracerebral contact. The stimulation may be performed using a periodic electrical signal (eg, with a frequency of about 1 Hz to 100 Hz inclusive), in particular using a current of about 0.1 mA to 20 mA inclusive.

The device may use high frequency electrical signals (eg at a frequency of about 400 kHz to 600 kHz inclusive) sent into the patient's brain, particularly in the vicinity of the at least one intracerebral contact point, in particular having a power of about 0.0001 W to 10W of power), treatment is performed by thermal coagulation.

### Intracerebral treatment or intracerebral function research methods

According to another aspect of the present invention, combined with the foregoing content, the present invention also aims to provide a method for studying and/or stimulating and/or treating brain functions, including the following steps:
step x: inserting in the patient's brain at least one multi-contact probe as described above for intracerebral functional studies and/or stimulation and/or treatment, in particular by radiofrequency,
step y: connecting said at least one probe to at least one recording and/or stimulation and/or treatment device,
Step z: Measuring neural brain activity and/or performing electrical stimulation in the patient's brain and/or these electrical treatments on the patient's brain by means of one or more intracerebral contacts.

### Brief description of the drawings

The invention will be better understood by reading here after, as examples and in a non-limitative way, in reference to the enclosed drawings on which:
[Fig. 1] Fig. 1 is a partial perspective schematic view of one example of a multi-contact probe for intracerebral function research and/or stimulation and/or treatment using radio frequency in particular, according to the present invention,
[Fig. 2] Fig. 2 is a partial schematic view of a cross section of a multilayer film portion forming the distal end portion of Fig. 1,
[Fig. 3] Fig. 3 is a perspective schematic view of one example of the distal end portion of the probe according to the present invention,
[Fig. 4] Fig. 4 is a perspective schematic view of another example of the distal end portion of the probe according to the present invention,
[Fig. 5] Fig. 5 is a perspective schematic view of another example of the distal end portion of the probe according to the present invention,
[Fig. 6] Fig. 6 is a perspective schematic view of another example of the distal end portion of the probe according to the present invention,
[Fig. 7] Fig. 7 is a perspective schematic view of another example of the distal end portion of the probe according to the present invention,
[Fig. 8] Fig. 8 is a partial perspective sectional view of a distal portion of an example of a probe according to the present invention,
[Fig. 9] Fig. 9 is an exploded view of Figure 8,
illustrate two different embodiments of an end-tip 400 of the probe 10a perspective schematic view of a proximal end of a distal end portion of an example of a probe according to the present invention,
[Fig. 10] Fig. 10 is a sectional view of a first example of an end-tip of the probe according to the present invention,
[Fig. 11] Fig. 11 is a sectional view of a second example of an end-tip of the probe according to the present invention,
[Fig. 12] Fig. 12 is a block diagram of an example of a method of manufacturing a probe according to the present invention,
[Fig. 13] Fig. 13 is a schematic view from above of one example of a multilayer film intended to form a probe according to the present invention,
[Fig. 14] Fig. 14 is a schematic diagram of one example of a multilayer film portion intended to form a distal end portion, viewed from above,
[Fig. 15] Fig. 15 is a view similar to Fig. 14 of another example of a multilayer film portion intended to form a distal end portion,
[Fig. 16] Fig. 16 is a view similar to Fig. 14 of another example of a multilayer film portion intended to form a distal portion and including two temperature sensors,
[Fig. 17] Fig. 17 is a separate schematic diagram of one temperature sensor in the example of Fig. 16, viewed from above,
[Fig. 18] Fig. 18 is a schematic diagram of a research device according to the present invention including a plurality of probes according to the present invention during use.

### Detailed description of the embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes.

Furthermore, elements that are identical or have the same function have the same reference numerals. For the sake of simplicity, they are not described in this specification with reference to each drawing, but only the differences between the embodiments.

Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

The term "comprising", used in the claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

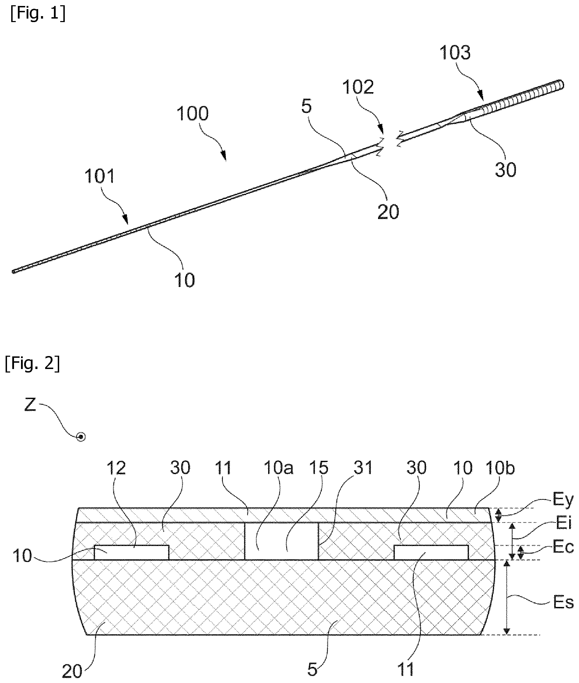

An example of a multi-contact neural probe 10 according to the invention for functional studies and/or stimulation and/or treatment in the brain, in particular using radiofrequency, is depicted in **Figure 1****.** The probe 10 includes a housing 100 with a distal portion 101, a proximal portion 103, and a connecting portion 102 connecting the distal portion 101 and the proximal portion 103 together.

The distal portion 101 has a cylindrical shape and is designed to be implanted in the patient's brain.

The proximal portion 103 has also a cylindrical shape and is intended to be connected to at least one recording and/or stimulation and/or treatment device external to the patient's body (not shown in this figure). The proximal portion 103 can have a larger diameter than the distal portion 101.

"Cylinder" or "cylindrical" refers to a surface with parallel straight line generators, that is, a surface composed of parallel lines in space. The end of the cylinder has two openings, each opening being adapted to be inscribed in a plane parallel to the other plane. The cross-section of the cylinder may take various shapes, such as circular, approximately circular, oval, elliptical, square, rectangular, star-shaped or other shapes. The cross-section of the cylinder forming the distal and proximal portions is preferably substantially circular.

The connecting portion 102 can have a relatively flat shape or a cylindrical shape or a conical or frustoconical shape.

The distal portion 101, the proximal portion 103 and the connecting portion 102 of the housing 100 are formed with a multilayer film 5 including a substrate 20 and at least one conductive layer 10 deposited on the substrate 20.

**Figure 2** illustrates in detail the multilayer film 5 from which the housing is formed.

In this example, the substrate 20 is made in one piece and includes at least one liquid crystal polymer material. In this example, the multilayer film 5 also includes an insulating layer 30.

As shown in FIG. 2, the conductive layer 10 forms a plurality of intracerebral contacts 11 and a plurality of transmission tracks 12 on the distal portion 101. The insulating layer 30 covers the transport track 12 so that the transport track 12 cannot come into contact with the patient's brain. In Figure 2, only a part of the transmission track 12 is shown for clarity of the drawing. In this example, each intracerebral contact 11 is associated with a transmission track 12.

In the example shown in FIG. 2, in addition to the substrate 20, the multilayer film 5 in the distal portion 101 also includes a first conductive layer 10a deposited directly on the substrate 20 and forming the transmission track 12. The first conductive layer 10a has a thickness Ec of 10 µm and an overall thickness Ei of 25 µm at the end 15 of the transmission track 12.

The multilayer film 5 further includes an insulating layer 30 also deposited on the substrate 20, the insulating layer 30 has a thickness Ei, and covers the entire substrate 20 that is not covered by the transport track 12 except for the end 15 of the transport track 12. The insulating layer 30 supports the undulations formed in the horizontal plane of the transport track 12. In this example, the or each insulating layer 30 includes a liquid crystal polymer material, such as the same material as the substrate 20.

As shown in FIG. 2, the multilayer film 5 includes a second conductive layer 10b with a thickness Ey of 1 to 40 µm, and preferably 10 µm, that is deposited on the insulating layer 30 and forms the intracerebral contact 11.

Each transport track 12 is in contact with an intracerebral contact 11 at its end 15. The intracerebral contacts 11 and the transmission track 12 are thus connected via through holes 31 in the insulating layer 30.

As described in more detail below, in the proximal portion 103 of the probe 10 the multilayer membrane 5 includes the substrate 20, at least one conductive layer 10 forming the transmission track 12, and connecting contacts 77 at the ends of the transmission track 12. The transport rail 12 is covered by at least one insulating layer 30. Connecting contacts 77 enable electrical contact between the probe 10 and external recording and/or stimulation and/or treatment devices.

Finally, in the connecting portion 102 of the probe 10, the multilayer film 5 includes a substrate 20, at least one conductive layer forming the transmission track 12, and at least one insulating layer 30 covering the transmission track 12.

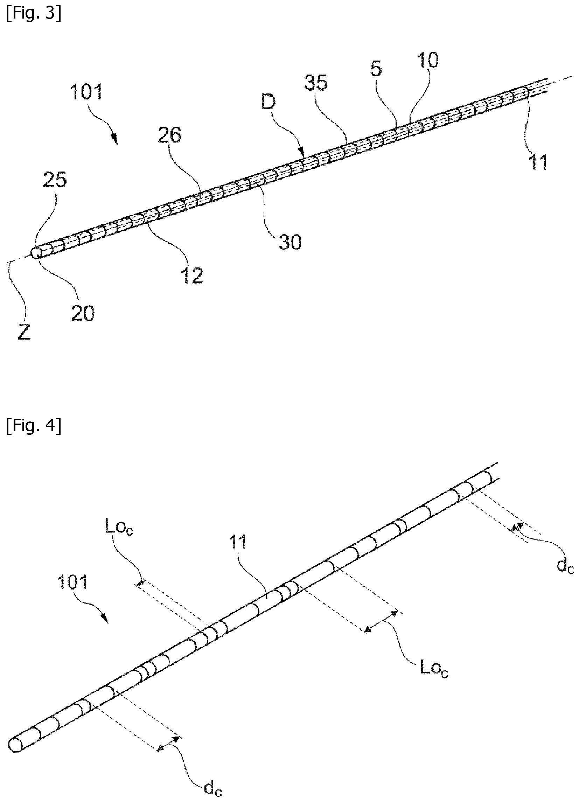

As shown in **Figure 3****,** the cylinder formed by the distal portion 101 defines an internal cavity, or lumen, 25 having a circular cross-section with an outer diameter D of approximately 0.8 mm. In this example, each intracerebral contact 11 extends over the entire circumference of the cylinder.

Furthermore, in this example, the distal portion 101 includes a temperature sensor 35 formed from the conductive layer 10.

Various examples of distal portion 101 of the probe 10 are depicted in **Figures 4-7****.** For reasons of clarity, the transport track 12 is not shown in these figures.

As shown in the example of Figure 4, the intracerebral contacts 11 have different lengths Lo_{C} and are spaced apart from each other by different distances d_{C}.

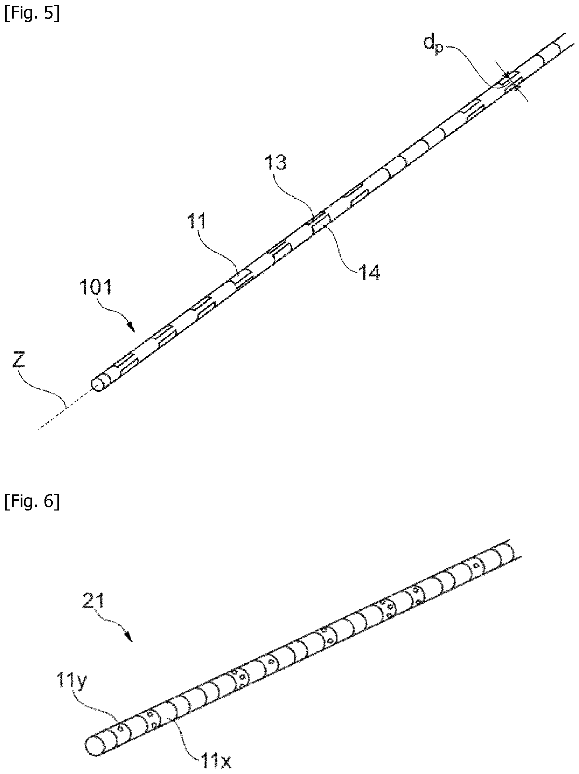

In the example of **Figure 5****,** the intracerebral contact 11 extends only over part of the circumference of the cylinder formed by the distal portion 101. Each intracerebral contact 11 extending transversely to the axis Z consists of two parts 13 and 14 separated from each other by a space of width dₚ. In this example, the spaces of width dₚ between portions 13 and 14 of a plurality of successively arranged intracerebral contacts 11 are not aligned with each other along the axis Z, but are offset.

In the example of **Figure 6****,** the distal portion 101 includes an intracerebral contact 11x extending over the entire circumference of the cylinder formed by the distal portion 101 and a circular-shaped intracerebral contact 11y.

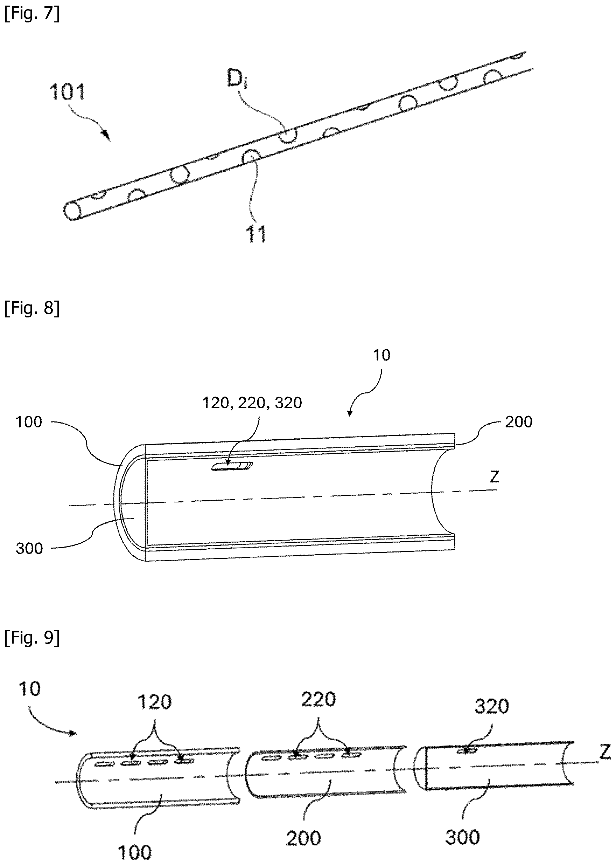

In the example of **Figure 7****,** the distal portion 101 includes a circular-shaped intracerebral contact 11 with a diameter Dᵢ equal to about 0,04 mm to 2,513 mm for a diameter of probe of 0,8 mm. Furthermore, the intracerebral contacts 11 are arranged in a five-point shape on the multilayer membrane 5.

The use of circular shaped intracerebral contacts 11 enables the creation of a probe 10 capable of generating directed pulses or measurements.

**Figure 8** presents a partial sectional view of the distal portion of the probe 10, and **Figure 9** is a exploded view of Figure 8.

As it is illustrated, the probe 10 comprises a housing 100, a tubular stiffener 200, and a catheter 300, the three of them coaxial to one another. The housing 100 defines an axial direction Z, extending between the distal portion 101 and the proximal portion 103, a radial direction, and a circumferential direction.

The catheter 300 is inserted inside the tubular stiffener 200 which is inserted inside the housing 100. Thus, the housing 100 is radially outside the tubular stiffener 200, and the catheter 300 is radially inside the tubular stiffener 200.

The housing 100 comprises a series of elongated openings 120 spaced apart from one another along the axial direction, and aligned along the axial direction. Each opening 120 of the housing 100 has an oblong shape elongated along the axial direction.

The tubular stiffener 200 also comprises a series of openings 220 corresponding exactly to the openings 120 of the housing 100. The tubular stiffener 200 is fixed inside the housing 100 so that its openings 220 face the openings 120 of the housing 100.

On the other hand, the catheter 300 comprises only a single elongated aperture 320. in this example, the elongated aperture 320 has the same shape and same dimensions as the openings 120 of the housing 100. In other embodiments, the elongated aperture 320 can have a different shape and/or different dimensions as the openings 120 of the housing 100. In particular the elongated aperture 320 might have bigger dimensions than the dimensions of the openings 120 of the housing 100. The catheter 300 is removably inserted into the assembly made from the housing 100 and the tubular stiffener 200, and can be moved inside it, at least in a rotational movement around its longitudinal axis, between a first position where the single aperture 320 of the catheter 300 matches an opening 120 of the housing 100, i.e. faces an opening 120 of the housing 100, and a second position where the single aperture 320 of the catheter 300 is away from any openings 120 of the housing 100, and thus faces a wall part of the housing 100.

In the first position, the probe 100 can deliver some liquid drug to an area of a brain facing the opening 120 of the housing. In the second position, the probe 100 is closed so as to provide any liquid, such as liquid drugs, from being delivered.

In the illustrated embodiment, the catheter 300 is closed at the tip of its distal end.

In a different embodiment, the probe 10 can be exempt of the tubular stiffener 200, i.e. comprising just the catheter 300 inserted directly inside the housing 100.

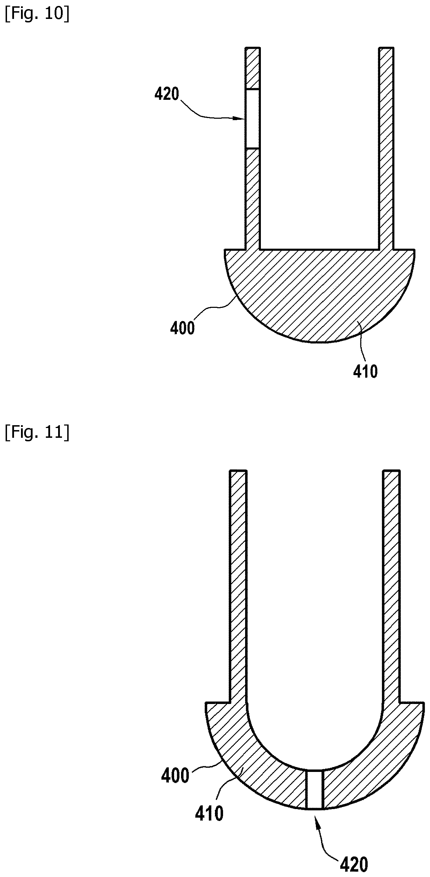

**Figures 10 and 11** illustrate two different embodiments of an end-tip 400 of the probe 10. The end-tips 400 illustrated in figures 9 and 10 can be either separate from the tubular stiffener 200 and inserted partially in the lumen 25 of the housing 100 though its distal end 71, or formed with the tubular stiffener 200 to form a single body.

In the embodiment illustrated in figure 9, the end-tip 400 can form a sealing plug with a bulk 410 having a smooth shape in order to enable a smooth insertion of the probe 10 inside the brain of a patient, and comprise eventually an opening 420 on a part facing an opening 120 in the cylindric wall of the housing 100, the opening 420 of the end-tip 400 being an opening 220 of the tubular stiffener 200 when the end-tip 400 and the tubular stiffener 200 are made together in one same material.

In the embodiment illustrated in figure 10, the end-tip 400 can form a sealing plug with a bulk 410 having a smooth shape in order to enable a smooth insertion of the probe 10 inside the brain of a patient, and comprise an opening 420 in its bulk 410 in order to deliver some liquid drug at the very end of the probe10 along its axial direction.

An example of a method of manufacturing a multi-contact neural probe 10 for functional studies in the brain according to the present invention is described in Figure 11, which method includes two steps a and b.

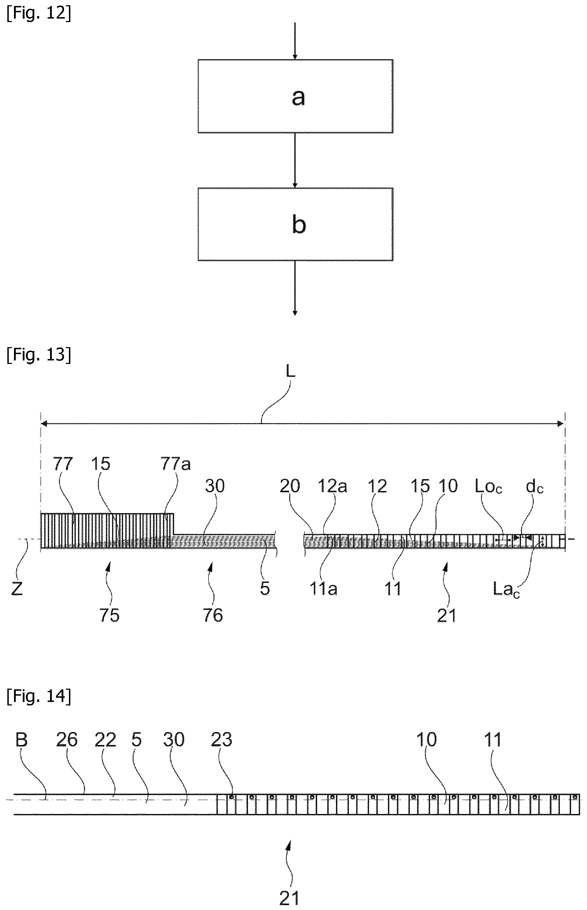

As shown in **Figures 12 and 13****,** in a first step a of producing the multilayer film 5, at least one conductive layer 10 is flatly deposited on a substrate 20 made of a liquid crystal polymer (LCP) material, in this example, where several conductive layers 10 are made of gold or have coatings of platinum and iridium.

In this example, the substrate 20 is cut out before step a, the length L of which is equal to approximately 400 mm. As shown in Figure 12, the substrate 20 is elongated, planar, and extends along the longitudinal axis Z. The substrate 20 has a first portion 21 intended to form the distal portion 101 of the housing 100 of a multi-contact probe 10 for functional studies in the brain, a portion 76 intended to form the connecting portion 102, and a second portion 75 intended to form the proximal portion 103 of the housing 100 of the multi-contact probe 10. As shown in FIG. 2, the thickness Es of the substrate 20 transversely to the axis Z is equal to 50 µm in this example.

In this example, various conductive layers 10 are deposited to form a plurality of intracerebral contacts 11 in the first portion 21, a plurality of connector contacts 77 in the second portion 75, and a plurality of transmission tracks 12. In this example, each intracerebral contact 11 is associated with a transmission track 12 and a connector contact 77.

In the depicted example, the conductive layer 10 is deposited in the manner described with reference to FIG. 2. The conductive layer 10 and the insulating layer 30 are deposited by stacking them on the substrate 20 and then compressed and heated.

Each intracerebral contact 11 is connected to a single connector contact 77 by a single transmission track 12. For example, the intracerebral contact 11a is connected to the connector contact 77a via the transmission track 12a.

In a second step b of the method according to the invention, a cylinder is formed around a catheter 300 extending along the longitudinal axis Z from the first part 21 of the multilayer film 5 to obtain a distal portion 101 of the probe intended for implantation in a brain of a patient to a second portion 75 of the multilayer film 5 to obtain the proximal end 103 of the probe 10 intended to be connected to at least one device for recording and/or stimulation and/or treatment outside the patient.

In the example shown in **FIG. 13**, the intracerebral contact 11 has a width La_{c} of 2.5 mm extending transversely to the axis Z over the entire width of the substrate 20 and a length Lo_{c} measuring 2 mm parallel to the axis Z. The intracerebral contacts 11 are identical to each other. The width of the transport track 12 is 50 µm. The intracerebral contacts 11 are regularly spaced apart from each other by a distance dc equal to approximately 1.5 mm.

In this example, the connector contacts 77 are deposited over a width greater than the length Lac of the intracerebral contacts 11. Connector contacts 77 are regularly spaced and have a constant length measured parallel to axis Z.

Various examples of a first portion 21 of a multilayer film 5 intended to form the distal portion 101 of the probe 10 after step a and before step b are depicted in FIGS. 14 to 16. For reasons of clarity, the transport track 12 is again not shown in these figures.

In **Figure 14****,** part 21 includes 18 intracerebral contacts 11 of the same size. The substrate 20 includes a window 23 therethrough on a lateral edge 22. The lateral edge 22 extends from a lateral end 26 of the substrate 20 to a portion of the substrate 20 close to this lateral end 26, in this example to line B.

In the example of **FIG. 14****,** step a is performed in such a way that the conductive layer 10 and the insulating layer 30 are deposited outside the window 23. Thus, the multilayer film 5 itself includes windows 23. The use of windows 23 may facilitate securing the lateral edges 22 together as described below.

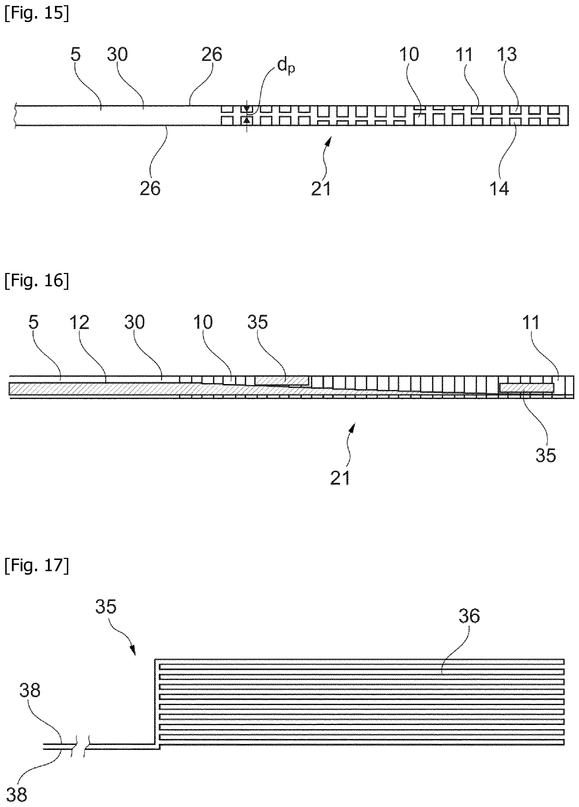

In the example of **FIG. 15****,** the intracerebral contact 11 does not extend over the entire width of the substrate 20 transversely to the axis Z, but only over a part of the width of the substrate 20. Each intracerebral contact 11 extending transversely to the axis Z consists of two parts 13 and 14 separated from each other by a space of width dₚ. Each portion 13 or 14 extends from a lateral end 26 of the substrate 20. In this example, the spaces of width dₚ between portions 13 and 14 of a plurality of successively arranged intracerebral contacts are not aligned with each other along the axis Z, but are offset.

In the example shown in **FIG. 16****,** on the first part 21 of the multilayer film 5, in addition to the intracerebral contacts 11 and the transmission track 12, two resistive temperature sensors 35 are formed, as shown in **FIG. 17** shown on a large scale.

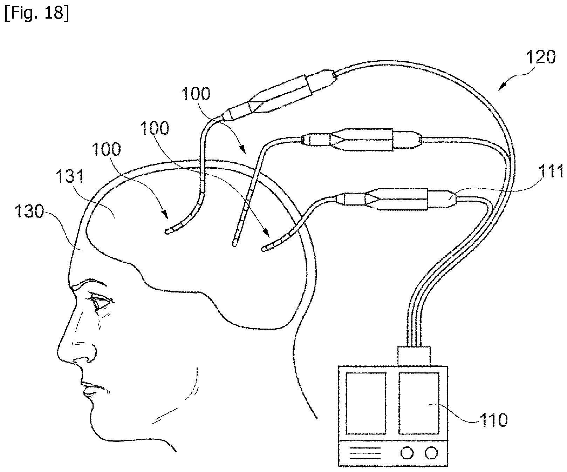

Each temperature sensor 35 includes a long zigzag track 36 connected to two tracks 37 and 38. As shown in Figure 18, the track 38 is connected to the transmission track 12, which is connected to the intracerebral contact 11.

The resistance of the circuit 39 between the track 37 and the transmission track 12 connected to the track 38 changes as a function of temperature.

As shown in **FIG. 18****,** the probe 10 can be connected by means of a connector 111 to a device 110 for recording and/or stimulating and/or treating a patient, to form a device for functional studies and/or stimulation of the brain, in particular by radiofrequency. and/or therapeutic multi-touch device 120.

The device 110 for recording and/or stimulating and/or treating a patient also enables processing of data received at the connector contact 77 transmitted from the intracerebral contact 11 by the transmission track 12.

As shown **in** **Figure 18****,** such a device 120 makes it possible to perform functional studies or treatments or stimulation within the brain, including the following steps:
Step x: Insert at least one multi-contact brain function research probe 10 as described above, in this example three probes 10, into the brain 131 of the patient 130,
step y: connecting the probe 10 to at least one recording and/or stimulation and/or treatment device 110, and
Step z: measure the brain electrical activity in the brain 131 of the patient 130 and/or perform electrical stimulation in the brain 131 of the patient 130 and/or perform electrical therapy on the brain 131 of the patient 130.

The invention is not limited to the embodiment just described.

In particular, the method may comprise a step of finishing the electrically conductive layer, such as an etching step.

The number of contacts within the brain may vary, for example from 2 to 500 (inclusive).

The conductive layer 10 may be deposited by some other process, in particular by a thin layer deposition process.

The multilayer film 5 can be rolled on itself in different ways, for example using more or fewer frustums.

The distal stud 70 may be connected via connecting wires to a device for recording and/or stimulating and/or treating the patient.

The various layers deposited on substrate 20 may have different thicknesses.

A plurality of conductive layers and/or insulating layers may be deposited on substrate 20, such as 2 to 10 conductive layers and/or insulating layers.

The insulating layer 30 may comprise some other polymer material, in particular some other biocompatible polymer material, such as polyamide.

In a variant not shown, the winding of the first part 21 of the multilayer film 5 is performed so as to partially overlap the lateral edges 22, for example by means of a frustum.

In case the rolling of the first part 21 of the multilayer film 5 is performed to partially overlap the lateral edges 22, the lateral edges 22 may be glued or welded together.

## Claims

1. A multi-contact neural probe for functional study and/or stimulation and/or treatment of a brain by radiofrequency, comprising:
- an elongated tubular housing with a proximal end, a distal end intended to be implanted inside a patient's brain, and an internal lumen forming a longitudinal passage inside the housing from the proximal end to the distal end,
- a multi-layer film arranged on an outer surface of said elongated tubular housing, said multi-layer film comprising:
o a polymer substrate and
o at least one conductive layer deposited on said substrate and including:
▪ one intracerebral contact arranged on said distal end,
▪ at least one connecting contact arranged on said proximal end and intended to be connected to at least one external device for recording and/or stimulating and/or treating, said at least one external device being external from a body of said patient, and
▪ at least one transmission track connected to said at least one intracerebral contacts and said connector contacts,
- at least one opening arranged on said distal end of said housing and allowing a fluid communication between the lumen and an aera facing said at least one opening, and
- a catheter designed to transport a liquid drug and having an aperture, said catheter being removably lodged inside the lumen and being movable inside the lumen between a first position where said aperture of the catheter faces one of said at least one opening of the housing in order to deliver some liquid drug and a second position where said aperture of the catheter is blocked by a portion of a wall of the housing in order to prevent any liquid drug delivery outside of the housing.

2. The multi-contact probe according to claim 1, further comprising a tubular stiffener inserted between the housing and the catheter, fixed onto the housing, and which has an opening for each opening of said housing, the or each opening of the stiffener facing an opening of the housing.

3. The multi-contact probe according to claim 1, further comprising an end-tip mounted on said distal end of the housing and closing said housing at said distal end.

4. The multi-contact probe according to claim 3 in combination with claim 2, wherein the tubular stiffener comprises said end-tip, said end-tip being formed in the same material bloc as the tubular stiffener.

5. The multi-contact probe according to claim 3, wherein an opening of said at least one opening of the housing is arranged in the end-tip.

6. The multi-contact probe according to claim 1, wherein said at least one opening arranged in the housing have an elongated shape along the longitudinal direction extending between the proximal end and the distal end.

7. The multi-contact probe according to claim 1, wherein said at least one opening of said housing is filled with a micro-porous material allowing a fluid to pass through.

8. The multi-contact probe according to claim 1, wherein the catheter comprises a locking system on a proximal end to realize a coupling with a tank or another tubular system.

9. The multi-contact probe according to claim 1, wherein said catheter comprises an integrated tank.

10. The multi-contact probe according to claim 2, wherein the tubular stiffener extends only along the distal end of the housing.

11. The multi-contact probe according to claim 1, comprising from 1 to 500 intracerebral contacts, in particular from 2 to 60 intracerebral contacts.

12. The multi-contact probe according to claim 1, wherein the catheter comprises at least one seal around its aperture on a radially outside face of the catheter.

13. The multi-contact probe according to claim 1, wherein the catheter comprises a single aperture.

14. The multi-contact probe according to claim 1, wherein the catheter comprises several apertures, at least two of said apertures being aligned along an axial direction of said catheter and sharing a same position along a circumferential direction, and said housing comprising two openings arranged to face said two apertures when the catheter is in its first position.

15. The multi-contact probe according to claim 1, wherein said multilayer film comprises at least one insulating layer of polymer material, preferably liquid crystalline polymer material, deposited on said substrate, the at least one insulating layer at least partially covers the at least one transmission track.

16. The probe according to any one of the preceding claims, wherein the substrate is made of at least one liquid crystal polymer (LCP) material or of polyimide.

17. Probe according to any one of the preceding claims, wherein said distal end comprises at least one temperature sensor formed by at least a portion of said at least one electrically conductive layer.

18. A multi-contact device for functional study and/or stimulation and/or treatment of the brain by radiofrequency, comprising a multi-contact probe, and at least one device connected said multi-contact probe for recording and/or stimulating and/or treating a patient, said multi-contact neural probe for functional study and/or stimulation and/or treatment of the brain by radiofrequency, comprising:
- an elongated tubular housing with a proximal end, a distal end intended to be implanted inside a patient's brain, and an internal lumen forming a longitudinal passage inside the housing from the proximal end to the distal end,
- a multi-layer film arranged on an outer surface of said elongated tubular housing, said multi-layer film comprising:
o a polymer substrate and
o at least one conductive layer deposited on said substrate and including:
▪ one intracerebral contact arranged on said distal end,
▪ at least one connecting contact arranged on said proximal end and intended to be connected to at least one external device for recording and/or stimulating and/or treating, said at least one external device being external from a body of said patient, and
▪ at least one transmission track connected to said at least one intracerebral contacts and said connector contacts,
- at least one opening arranged on said distal end of said housing and allowing a fluid communication between the lumen and an aera facing said at least one opening, and
- a catheter designed to transport a liquid drug and having an aperture, said catheter being removably lodged inside the lumen and being movable inside the opening between a first position where said aperture of the catheter faces one of said at least one opening of the housing in order to deliver some liquid drug and a second position where said aperture of the catheter is blocked by a portion of a wall of the housing in order to prevent any liquid drug delivery outside of the housing.

19. A method of manufacturing a multi-contact probe for functional study and/or stimulation and/or treatment of the brain by radiofrequency as claimed in any one of the preceding claims, comprising the following step:
a) Step a: forming a multilayer film by flatly depositing at least one conductive layer on at least a portion of the substrate, said at least one conductive layer forming at least one intracerebral contact, at least one connector contact and at least one transmission track, each transmission track being connected to the intracerebral contact and the connector contact,
b) Step b: forming a cylinder around a catheter extending along the longitudinal axis from at least a first portion of the multilayer film to obtain the distal portion to a second portion of the multilayer film to obtain the proximal portion.
